# EUROPÄISCHE PATENTANMELDUNG

(11) **EP 0 985 413 A1**
(43) Veröffentlichungstag der Anmeldung: **15.03.2000**
(21) Anmeldenummer: 98114781.2
(22) Anmeldetag: 06.08.1998
(51) Int. Cl.: A61K 9/70, A61L 29/00

(54) **Medizinprodukte mit retardierter pharmakologischer Aktivität und Verfahren zu ihrer Herstellung**

(71) Anmelder: Schierholz, Jörg Michael, Dr. Dr., 51427 Bergisch Gladbach (DE)
(72) Erfinder: Schierholz, Jörg Michael, Dr. Dr., 51427 Bergisch Gladbach (DE); Pulverer, Gerhard, Prof. Dr., 50828 Köln (DE)
(74) Vertreter: Meyers, Hans-Wilhelm, Dr.Dipl.-Chem.

(57) **Zusammenfassung**

Medizinprodukt umfassend mindestens zwei Substanzen, von denen eine erste als Substanz A und eine zweite als Substanz B bezeichnet wir und Substanz A eine Löslichkeit (w/w) in Wasser von 1:1.000 bis 1:1.000.000 aufweist, Substanz B eine höhere Löslichkeit aufweist als Substanz A und zumindest eine der Substanzen A und B ein pharmazeutischer Wirkstoff ist.

## Beschreibung

Gegenstand der vorliegenden Erfindung sind Medizinprodukte mit retardierter pharmakologischer Aktivität sowie Verfahren zu ihrer Herstellung.

DE-A-36 13 213 offenbart ein resorbierbares poröses Tricalciumphosphat mit einer Mischung aus einem Antibiotikum und einem weiteren Füllstoff, insbesondere einer hydrophilen Aminosäure, als besonders vorteilhaft bei der Herstellung von Knochenzementen. Hierbei wird ein niedermolekularer Zusatz in das Tricalciumphosphat hineingegeben: Glycin, welches wasserlöslich ist und welches die Wirkstoffabgabe aus dem porösen Tricalciumphosphat beschleunigt.

WO 84/03623 offenbart Magnesiumoxid, -hydroxid oder andere Magnesiumsalze, welche Effekte auf die Freisetzung von Tabletten mit Calciumantagonisten haben.

WO 89/09626 offenbart einen Hydrogel-beschichteten Schlauch, der mit Polymyxin beladen wird und verhindern soll, daß Polymyxin-sensitive Mikroorganismen Thromben formieren oder eine Infektion induzieren.

US-P-4,612,337 beschreibt eine Methode, in der Kunststoffmaterialien in Lösungen mit verschiedenen Wirkstoffen eingelegt werden, beispielsweise Ethanol, Chloroform, und später die somit oberflächlich beschichteten Materialien getrocknet werden und ein Short-term-release an Wirkstoffen induzieren.

EP-A-0 520 160 beschreibt das Quellen von Polyurethankathetern mit fluorierten Kohlenwasserstoffen, in welche durch solch einen Prozess quarternäre Ammoniumsalze inkorporiert werden. Nach Trocknung sollen die Katheter durch Freisetzung antimikrobiell wirksam werden.

DE-A-37 25 728 beschreibt ein Verfahren, bei dem Silbermetallionen in Polyurethansilikon-Mischungen gegeben werden. Hierbei soll durch die Mischung von Polyurethanen mit Silikon Körperflüssigkeit in das Innere der Kunststoffmischung gelangen und so die Silberionen herauswaschen, welches einen besseren Keimtötungseffekt erzielen soll.

WO 89/04628 offenbart die Behandlung von Silikonimplantaten mit Antibiotika und Chloroform derart, daß die Wirkstoffe in die äußeren Schichten des Implantatmateriales hineindiffundieren können und somit für eine kurzfristige Abgabe an Wirkstoff nach Implantation des fertigen Devices gesorgt wird.

WO 87/03495 beschreibt genau das gleiche Verfahren wie WO 89/04628, mit einer Zeichnung, wie Katheter imprägniert werden können.

EP 0 484 057 A2 beschreibt einen wäßrigen Aufstrich, mit welchem Medizinprodukte antithrombogen werden sollen, indem organische Polymere, Bindungsstoffe, Spacer und Heparin als antithrombogenes Agens mit Methylenacrylsäure-Copolymer und kolloidalem Silikaten vermischt werden. Dieses Harz kann auch vorher sulfoniert werden, bevor dieser Harzanstrich auf die Medikalprodukte aufgebracht wird.

US-P-4,642,104 offenbart einen Urethralkatheter aus einem olefinischen Polymer oder einem Silikonmaterial, in welchem antimikrobielle Substanzen außen und innen gebunden werden.

US-P-4,677,143 beschreibt antimikrobielle Mischungen, mit welchen Medizinprodukte beschichtet werden. Diese Mischungen bestehen aus Acrylnitritblock-Polymeren, Polyvinylchlorid, Mischungen von Polyestern und Polyurethanen, Styrolblock-Copolymeren und natürlichen Gummiarten und Polycarbonaten, Nylon und Silikonmaterialien, welche mit oligodynamischen Substanzen, also antibakteriellen Metallen, vorzugsweise Silberoxidverbindungen gemischt werden. Diese Katheter wurden in einer klinischen Prüfung mit 1.300 Patienten untersucht. Es wurde kein Unterschied zu unbeschichteten Kathetern gefunden (Riley et al., 1995, Am. J. Med.).

WO 87/01595 offenbart, wie antimikrobielle Wirkstoffe, Antibiotika zu Plastikmaterialien vor dem chemischen Polymerisationsprozess hinzugegeben werden. Hier wird der Wirkstoff vor der Polymerisation gleichförmig in dem Kunststoff verteilt. Anwendungsbeispiel: Fixateur Externe.

WO 95/04568 beschreibt Injection Ports, welche antimikrobielle Substanzen beinhalten, beispielsweise Chlorhexidin, Silbersulfadiazin und andere.

US-P-5,525,348 offenbart Beschichtungen mit Polyvinylpyrrolidon in unterschiedlichen Lösungsmitteln, worauf verschiedene andere Oligomere mit Heparinbenzalkoniumchlorid hinzugegeben werden, die ionische Surfactant-Beschichtungen ergeben.

EP 0 640 661 A2 beschreibt Mischungen von Silikon mit silberenthaltenden Zeoliten, wobei die Silberionen aus diesen Zeoliten diffundieren.

EP 0 301 717 B1 beschreibt ebenfalls Zeolite, die in medizinische Kunststoffe eingearbeitet werden. Diese Zeolite beinhalten Silber, Kupfer oder Zink, welche eingeknetet werden und den antimikrobiellen Effekt erzielen sollen.

EP 0 470 443 B1 offenbart, wie bei niedrigen Extrusionstemperaturen dünne Coatingschichten beispielsweise auf Kathetermaterialien zusammen mit temperatursensiblen pharmazeutischen Inhaltsstoffen aufgebracht werden.

US-P-5,451,424 beschreibt Laminationsverfahren über Extrusion von Kunststoffen, mit welchen medizinische Produkte mit Arzneimitteln außen und innen beschichtet werden.

WO 92/01542 beschreibt, wie über "Solvent Casting", d.h. zur Lösung von Kunststoffen und dem gleichzeitigen Lösen von Arzneimitteln und das Eintauchen der Medikalprodukte, Beschichtungen für Nasoothopharyngialtuben hergestellt werden, beispielsweise mit dem Zweck, chronische Infektionen und Inflammationen des umgebenden Gewebes zu verhindern.

WO 96/39200 beschreibt, daß mit "Solvent Casting" ein Metallsalz in eine Kunststoffmatrix inkoorporiert werden kann.

US-P-4,999,210 beschreibt, wie eine homogene Schmelze aus Kunststoffen und Chlorhexidin hergestellt wird und über einen 2-Schrauben Compounder die Mischung auf medizinische Artikel koextrudiert wird. Später werden diese Artikel noch einmal in eine Dipping-Lösung (solvent casting) mit einem hydrophilen Polymer gegeben.

EP 0 350 161 A2 offenbart, wie mit einem Ammoniumsalz ein Substrat beschichtet und dann bei alkalischem pH-Wert heparinisiert wird.

WO 91/01767 beschreibt, wie über "Solving Casting", also (Dichlor-methan) Isopropanol und anderen Lösungsmitteln, Ammoniumheparinkomplexe an medizinische Kunststoffe gebracht werden.

EP 0 231 573 B1 beschreibt, daß pharmazeutische Substanzen entweder bei Solving Casting oder Adsorption durch Quellung in feste Kunststoffe inkoorporiert werden.

US-P-5,514,673 beschreibt eine pharmazeutische Zusammenstellung, in der über eine *transmuskosale* Applikation von Progesteron von 17-Beta-Estradiol klinische Effekte erzielt werden. Hierbei werden 3 µg bis 0,5 mg 17-Beta-Estradiol bzw. Progesteron mit natürlichen Ölen vermischt und Ei-Lecithin hinzugegeben. Diese Mischungen sollen über die Nase appliziert werden und dort Effekte ausüben. Mit dieser Methode sollen Hautirritationen vermindert werden, wobei das Lecithin als Löslichkeitsvermittler wirkt.

WO 96/32907 beschreibt einen metallischen Stent mit einer hydrophoben elastomeren Kunststoffschicht, in welche eine biologisch aktive Substanz inkoorporiert ist. Die Methode beinhaltet das "Solvent Casting". Heparin ist hier bevorzugt mit 10 bis 45% Gewicht, mit einer Schichtdicke zwischen 30 und 150 µm, bzw. Dexamethason mit einem Anteil zwischen 0,5 und 10%.

WO 96/39215 beschreibt, wie ein Silikonschlauch aufgebaut wird, welcher im Inneren des Materials eine Schicht mit pharmakologisch aktiven Reagenzien aufweist, welche langsam durch die nicht-beschichtete Außenschicht nach außen diffundieren. Über eine Silikonextrusionsmaschine wird ein Sandwich-Katheter aufgebaut. Rifampicin und Minocyclin werden hier als Puder in die Zwischenschicht eingegeben.

WO 96/33670 offenbart, wie antimikrobielle Mischungen, vorzugsweise Rifampicin, Minocyclin-Mischungen über Quellung und Erhöhung der Temperatur mit anschließender Alkalisierung der Lösung in medizinische Kunststoffe eingebracht werden.

WO 94/10838 beschreibt Lösungen mit Minocyclin-EDTA, mit denen Medikalprodukte gespült werden, um damit Infektionen zu verhindern.

US-P-5,688,516 beschreibt Mischungen aus pharmazeutischen Substanzen, welche die oben genannten drei Gruppen an Wirkstoffen enthalten sollen: Antikoagulantien, Antithrombotika und Komplexbildner wie EDTA oder DMSA. Diese Wirkstoffe sollen auf über TDMAC vorbeschichtete Medizinprodukte gebracht werden.

Allogene Implantmaterialien sind essentiell in der modernen Medizintechnik. Die Vorteile, die mit diesen Medizinprodukten erreicht werden, also Medizinprodukten wie Peritonealkathetern, kardiovaskularen Kunststoffprodukten, orthopädischen Implantaten oder anderen prothetischen Implantaten, werden durch infektiöse Komplikationen oder thrombotische Ereignisse oder andere sekundäre Nebenwirkungen beeinträchtigt. Bei Infektionen von Kunststoffimplantaten sind die hauptverursachenden Infektionserreger Staphylococcus epidermidis und Staphylococcus aureus. Bei Gefäßkathetern sind diese Keime für 70 bis 80% aller Infektionen verantwortlich. Candida albicans ist für 10 bis 15% aller Katheterinfektionen verantwortlich. Bei Infektionen des harnableitenden Systems (UTI = Urinary Tract Infections) geht man davon aus, daß der Gebrauch von urinableitenden Kathetern wie Foley-Kathetern, suprapubischen- und Nephrostomie-Kathetern mit der Mehrzahl dieser Infektionen assoziiert ist. Solche Katheter werden häufig in ältere Patienten, Patienten mit einer Lähmung des Urogenitaltraktes, in postoperative Patienten und solche mit einer obstruktiven Uropathie implantiert. Trotz der Berücksichtigung aller sterilen Kautelen bei Insertion und Offenhaltung all dieser Katheter bleiben diese Infektionen ein größeres Problem. In UTI sind gramnegative Bakterien mit 60 bis 70% die Hauptverursacher der Infektionen, gefolgt von Enterokokken und Candida. Im Falle metallischer Prothesen ist aufgrund der fortgeschrittenen Operationstechnik die relative Zahl an Infektionen sehr viel geringer als bei Kathetermaterialien. Da aber diese Infektionen nur durch eine Explantation der infizierten Materialien zu sanieren und die Kosten für Sanierung und Reimplantation sowie die Mortalität bei älteren Patienten sehr hoch sind, sind präventive Maßnahmen ökonomisch und medizinisch betrachtet unabdingbar. Ähnliches gilt für Gefäßprothesen; hierbei rechnet man mit Infektionsraten zwischen 1 und 3% bei inguinalen Gefäßprothesen, welche zudem nach Infektion einer bestehenden Prothese mit wesentlich höheren Infektionsraten reimplantiert werden.

Hier besteht das Problem in der hohen Mortalität mit bis zu 50% der Patienten und der hohen Amputationsrate der distal betroffenen Extremität. Ein weiteres Problem besteht bei allen Medikalprodukten wie zentralen Venenkathetern, intrakoronalen Stents, Herzklappen (Olenoff (1)), resorbierbaren Wundauflagen, CAPD-Kathetern, Spinnvliesen, oder auch Gefäßprothesen in der Thrombogenität. Die thrombogene Potenz von Fremdkörpermaterialien läßt sich grundsätzlich durch die Modifikation mit antithrombogenen Arzneimitteln verringern. Eine dritte Gruppe von Indikationsfeldern betrifft okulare Implantate, wobei hier antiinfektive und antihypertensive Mittel als Beschichtungssubstanzen vorteilhaft sind. Andere Indikationsfelder sind die Verhinderung der Inkrustation durch Komplexbildner- Freisetzung aus UT-Kathetern und die kontrollierte Abgabe von Hormonen, Antihormonen, Zytostatika und Peptiden aus Zementen, Prothesenmaterialien, Herzschrittmachern, Stents und andere Materialien.

Eine bekannte Methode zur Modifikation von Medikalprodukten beruht darauf, daß auf die Oberfläche von Kathetern eine Schicht mit Tridodecylmethylamoniumchlorid (TDMAC) -surfactant und darauffolgend antimikrobielle Substanzen aufgebracht werden, welche ionisch geladen sind. Beispielsweise können Polyethylen-Silikon-Elastomere, Polytetraflourethylen oder auch Dacronmaterialien in 5%igen TDMAC-Lösungen für 30 Minuten gebadet, getrocknet und gespült werden. Diese TDMAC-Surfactant tragende Medizinprodukte können dann in verschiedene Wirkstofflösungen inkubiert werden.

Ähnliches kann man mit Benzalkoniumchlorid und der ionischen Bindung von antimikrobiellen Substanzen durchführen (Solomon DD, Sheretz, REJ in J. Contr. Release 6:342-352 (1989) und US-Patent Nr. 4,442,133).

Andere Methoden beinhalten ein ionisches Binden von Wirkstoffen mit negativ geladenen Gruppen an der Oberfläche von Medikalprodukten (US-Patent Nr. 4,895,566),
- das Anquellen von Medizinprodukten und die Adsorption in die Oberfläche (US-Patent Nr. 4,917,686),
oder das Aufbauen von ionischen Hydrogelen, welche dann später ionisch gebundene Wirkstoffe tragen (US-Patent Nr. 4,107,121)
- bzw. das Laminieren von antimikrobiellen Substanzen an oberflächliche Schichten (US-Patent Nr. 5,013,306)
- oder auch nur die Anwendung eines Silikonöls auf ein Medizinprodukt und dann Inkontaktbringung des Silikonfilmes mit Wirkstofflösung (US-Patent Nr. 4,952,419).

Ein Nachteil dieser Methoden liegt darin, daß durch solche physiko-chemischen Methoden die Wirkstoffaktivität relativ schnell nachläßt. Hinsichtlich der antimikrobiellen Wirksamkeit ist es bespielsweise so, daß nach dem Absinken der bakteriziden Oberflächenaktivität Katheter oder Gefäßprothesen oder andere Implantate wieder bakteriell besiedelt werden können. Gleiches gilt für die Beschichtung mit antithrombogenen Mitteln. Hierbei ist klinische Effizienz nur dann zu erwarten, wenn in ausreichender Menge Wirkstoffe freigesetzt werden. Ähnliches gilt auch für antiproliferative und proliferative Substanzen. Nachteil dieser Beschichtungen ist die fehlende effiziente Kontrolle der Freisetzung der pharmazeutischen Substanzen aus den Medizinprodukten (4). Wünschenswert ist es, einer idealisierten Freisetzung nullter Ordnung sehr nahe zu kommen, um über längere Zeit hinweg konstante Wirkungen zu erzielen.

Auch Gefäßprothesen und metallische Implantate (z.B. TEP's) können mit oder ohne Spacer (Albumin, Kollagen, biodegradierbare Coatings) mit antibakteriellen, antithrombotischen, antiflammatorischen, immunmodulierenden, endokrin wirksamen Substanzen modifiziert werden.

Aufgabe der Erfindung ist die Schaffung von Medizinprodukten, welche pharmakologisch aktiv sind, sowie Methoden bereitzustellen, mit denen möglichst lange eine pharmakologische Aktivität aufrechterhalten werden kann und so die genannten Nachteile des Standes der Technik zu überwinden. Eine weitere Aufgabe der Erfindung ist es, eine praktische, kostengünstige, sichere und effiziente Methode zur Verfügung zu stellen, um eine Vielzahl von Kathetertypen und andere Medikalprodukte mit unterschiedlichsten Substanzkombinationen zu modifizieren. Ein Ziel dieser Erfindung liegt u.a. darin, Medizinprodukte möglichst lange gegen Infektionen zu schützen, und dies mittels unterschiedlicher Inkorporierungs- und Beschichtungsmethoden.

Die Aufgabe wird gelöst durch Medizinprodukte enthaltend eine Substanz A und eine Substanz B, wobei Substanz A eine Löslichkeit (w/w) in Wasser von 1:1.000 bis 1:1.000.000 aufweist, Substanz B eine höhere Löslichkeit aufweist und zumindest eine der Substanzen A und B ein pharmazeutischer Wirkstoff ist. Dabei kann eine geringe Löslichkeit sowohl kinetisch als auch thermodynamisch bedingt sein.

Die Löslichkeit ergibt sich durch die maximale Menge einer Substanz, die bei 25°C in Wasser löslich ist. Berechnet wird das Gewicht der Substanz bezogen auf das Gesamtgewicht der erhaltenen Lösung. Im Rahmen dieser Anmeldung werden geringlösliche Substanzen auch mit der Eigenschaft "lipophil" charakterisiert.

Geeignete Medizinprodukte sind beispielsweise Medizinprodukte metallischer und nicht-metallischer Form wie Endoprothesen, Knieprothesen oder auch Katheter und Drainagen und andere polymere Medizinprodukte, welche mittels unterschiedlicher Methoden mit einer Kombination pharmazeutischer Substanzen bzw. Wirkstoffe modifiziert werden. Durch die Modifizierung mit pharmazeutischen Substanzen infektionsresistenter werden sollen oder b) hämo- oder biokompatibler oder c) letztendlich die Funktion des Medikalproduktes bzw. das Einwachsen in das umgebende Gewebe verbessern bzw. andere spezifische pharmakologische Effekte erzielen sollen. Wesentlich ist die Kombination mindestens zweier unterschiedlicher Substanzen für eine Beschichtung, Imprägnierung oder anderweitige Modifikation von Medizinprodukten, wobei eine Substanz eine geringe Löslichkeit in Wasser aufweist und die zweite Substanz eine höhere Löslichkeit in Wasser hat. Dies führt dazu, daß die Substanz A in dem umgebenden Medium (Liquor, Blut, Urin, Gewebe oder Peritonealflüssigkeit) eine geringere Löslichkeit aufweist als im Polymeren bzw. so schwer löslich ist, daß durch die Lösungsprozesse in unterschiedlichen Körperflüssigkeiten eine lange Freisetzungsphase ermöglicht wird, wie es auch bei nur oberflächlich beschichteten Totalendoprothesen der Fall sein kann. Die gering lösliche Substanz A sorgt überraschenderweise dafür, daß die Freisetzung der anderen Substanz B retardiert, d.h. verlangsamt wird. Durch die Verlangsamung der Freisetzung der Substanz B erhält man ein sogenanntes "steady state release"-Profil, in welchem man nach einem sehr flachen initialen Abfall der Freisetzung eine nahezu konstante Freisetzung erzielt. Die solchermaßen modifizierten Medizinprodukte sind, insbesondere wenn Substanz B ein Wirkstoff ist, in der Lage, länger pharmakologisch aktiv zu bleiben.

Die Erfindung wird im folgenden am Beispiel einer auf eine Substanz A und eine Substanz B beschränkten Kombination erläutert. Es ist für den Fachmann verständlich, daß auch mehrere Substanzen aus den jeweiligen Gruppen vorliegen können.

Dadurch, daß die Substanz A in dem späteren Implantationsgewebe (Blut, Liquor, Urin, Peritonealflüssigkeit, Gewebsflüssigkeit) nur sehr gering löslich ist, erhält man eine retardierte, verlängerte Freisetzung dieser Substanz. Das Überraschende ist, daß durch die verlangsamte Freisetzung dieser Substanz A auch die Freisetzung einer normalerweise sehr viel schneller abgegebenen Substanz B verzögert wird. Die Substanz A wirkt erstens überraschenderweise als Eigendepot, d.h. als Reservoir, welches sich nach und nach sehr langsam auflöst, und zweitens überraschenderweise als Diffusionsmatrix für Substanz B, die ohne die diffusionsbezogene Matrix sehr viel schneller in kurzer Zeit abgegeben werden würde. Die Löslichkeit der Substanz A darf jedoch nicht so gering sein, daß die Substanz A praktisch unlöslich ist, da dann keine pharmakologisch wirksamen Konzentrationen im umgebenden Medium erreicht werden. Ungeeignet ist beispielsweise Silberchlorid. Es kann sowohl nur eine der Substanzen A und B ein Wirkstoff sein als auch - bevorzugt - beide Substanzen. Bevorzugte Wirkstoffe weisen antimikrobielle, antithrombogene antiproliferierende, immunmodulierende und/oder endokrine Eigenschaften auf. Es hat sich bewährt, daß eine Beladung von mindestens 0,1% Masseprozent Substanzen in der Wirkstoffträgerschicht vorhanden ist, um über einen längeren Zeitraum pharmakologische Wirkungen in der Mikroumgebung des Medizinproduktes zu erzeugen. Bei der Mischung der Subtanz A mit Substanz B hat es sich bewährt, daß die Substanz A einen Anteil größer als 0,1% (Größenordnung) der gesamten Substanz hat. Sehr viel geringere Anteile an der Kombination haben nicht mehr die gewünschte Retardierungswirkung. Die Retardierungswirkung steigt in der Regel mit steigender Inkorporationsrate und Anteil der Substanz A.

Es ist bevorzugt, daß der Wirkstoff in wäßrigen Medien keine hohe Löslichkeit aufweist, also lipophil ist. Eine Lipophilisierung von hydrophilen Wirkstoffen kann - falls das Trägergerüst nicht ausreichend lipophil ist - dadurch erreicht werden, daß die Wirkstoffe durch kovalente oder nicht-kovalente Modifikation lipophilisiert werden, wie beispielsweise durch Veresterung, Veretherung, Acetal- oder Halbacetalbildung, Ringbildung, Adduktbildung oder in nicht-kovalenter Form mittels Komplexbildung, Salzbildung. Zum Beispiel kann Gentamicin-Salz, Gentamicin-Base mit einer lipophilen Fettsäure modifiziert werden, um somit die Lipophilie zu erhöhen. Gleiches gilt für Erythromycin-Stearat oder auch Clindamycin-Palmitat und viele andere pharmazeutische Substanzen, deren Galenik somit verändert werden kann.

Es wird bevorzugt, daß sich der Wirkstoff intrazellulär in den Zielorganen oder Organismen (z.B. Bakterien, Thrombozyten) anreichert. Als Beispiele sind aufzuführen die intrazelluläre Anreicherung von Rifampicin oder Erythromycin, Fusidinsäure, Imidazol oder Clindamycin-Derivaten in Baktieren. Gleiches gilt für das lipophile Mupirocin und Chinolon-Derivate. Ähnliches gilt aber auch für lipophile Wirkstoffe wie Ticlopidin, welche sich in Thrombozyten anreichern können.

Es ist besonders bevorzugt, daß Substanz A und B beides Wirkstoffe sind, die sich intrazellulär anreichern.

Geeignete Medizinprodukte sind Augenlinsen, Katheter, Gefäßprothesen, Endoprothesen, chirurgisch antimikrobielle Wirkstoffträger wie Kollagenvlies, Stents, Blades, Knochenzement, metallische Endoprothesen, Knieprothese, Hüftprothese, CAPD-Katheter, Wundauflagen, gesprühte Polyurethanvliese, Drainage, Knochen-Weichgewebeersatzstoffe.

Bevorzugte Materialien für die Medizinprodukte sind Siloxane, Polyurethane, Acrylate, Polycarbonate, Cellulose, Cellulose-Derivate, Polymere aus Tetrafluorethylen und Polyethylentherephthalat.

Gegenstand der Erfindung ist weiterhin ein Verfahren zur Steuerung der Freisetzung einer Substanz B aus einem Medizinprodukt, bei dem Substanz B zusammen mit einer Substanz A im Medizinprodukt enthalten ist, wobei Substanz A eine Löslichkeit (w/w) in Wasser von 1:1.000 bis 1:1.000.000 aufweist, Substanz B eine höhere Löslichkeit aufweist als Substanz A und zumindest eine der Substanzen A und B ein pharmazeutischer Wirkstoff ist.

Die Substanzen können auf unterschiedlichste Art und Weise mit den Medizinprodukten kombiniert werden. Eine hocheffiziente Modifikation von Medizinprodukten findet über Quellung mittels geeigneter Lösemittel und geeigneter Quellpartner statt wie in EP 0 550 875 beschrieben. Hierbei werden aufgrund ähnlicher Löslichkeitsparameter (Hansen Parameter, Kohäsionsenergiedichten) von Polymer und pharmazeutischer Substanz ausreichend hohe Konzentrationen an Wirkstoffen in Medizinprodukten erreicht. Diese so beladenen Medizinprodukte werden mittels kontrollierter Freisetzung der Wirkstoffe aus diesen Medizinprodukten heraus pharmakologisch wirksam.

Beispiele hierfür sind die Quellung von Polyurethanen mit Alkoholen, Aceton, Ketonen, Ester wie Essigsäureethylesther, bei ausgesuchten aromatischen Polyurethan mit CH₂Cl₂, DMF, DMA, Methylethylketon, HMPT, DMSO, Dioxan (heiß), n-Cresol, Tricresol, n-Propanol, Dichloressigsäure oder Mischungen davon.

Beispiele für geeignete Kombinationen von pharmazeutischen Wirkstoffen, Quellmitteln und Polymeren können der folgenden Tabelle entnommen werden.

| Substanz | Quellmittel | Polymer |
|---|---|---|
| Cyclophosphamid | Ethanol, Chloroform, Toluol, Aceton, Dioxan | PUR, PDMS, Poly-Polycarbonat, Polyethylen |
| Tamoxifen | Chloroform, Hexan | PDMS, PMMA, Polyethylen |
| Buselerin | Methanol, Ethanol | PUR, Polycarbonat, Cellulose |
| Ketoconazol | Chloroform, Methanol | PDMS, PMMA, Polycarbonat, Polyethylen |
| Diclofenac | DMF, Ethanol | PUR, Polycarbonat |
| Indometacin | Chloroform | PMDS, PMMA |
| Ibuprofen | Chloroform, Ethanol, Aceton, Ether | PMDS, PMMA, Teflon |
| Piroxicam | Methanol | PUR, Polycarbonat, Cellulose |
| Phenylbutazon | Chloroform, Aceton | PDMS, PMMA |
| Acetylsalicylsäure | Ethanol | PMMA, Polyester |
| Glukokortikoide (Steroide) | Ethanol, Methanol, Acton, Chloroform mit Ausnahme von Triaminolonacetonid, welches gut in DMF löslich ist | Zur Quellung können fast alle verwendbaren Polymere im Bereich mittlerer Kohäsionsenergiedichten verwendet werden, da es sich bei den Steroiden um amphiphile Stoffe handelt |
| Acetylsalicylsäure | Ethanol | PUR, PMMA, Polyester |
| Isosorbidnitrat | Chloroform, Aceton | PDMS, Teflon, PMMA |
| Nifedipin | Chloroform, Aceton | PDMS, Teflon, PMMA |
| Verapamil | Chloroform, Aceton, Ethylacetat | PDMS, Teflon, PMMA, Polyethylen |
| Nadolol | Methanol | PUR, Polycarbonat, Cellulose, Polyethylenterephthalat |
| Metropolol | H₂O, Ethanol | Cellulose, PUR |
| Pindolol | höhere Alkohole | PUR, Polycarbonat, Cellulose |

Weitere Informationen zur Auswahl geeigneter Partner sind in der EP-A-0 560 875 enthalten.

Auch Polyethylentherephthalat kann mit ausgesuchten Solventien, Aceton, Benzaldehyd, Dichlorethan, Diphenylether, Acetophenyl, THF, Dioxan, CHCl₃, CH₂Cl₂ in den Quellungszustand überführt werden. PET wird beispielsweise für künstliche Gefäße und Herzklappen eingesetzt.

Eine andere effiziente Methode beruht darauf, daß mit Hilfe von Lösungsmitteln Polymere aufgelöst, mit den pharmazeutischen Produkten vermischt, in eine Form gegeben werden und das Lösemittel evaporiert wird; dies nennt man "Solvent Casting". Die Technik des Solvent Casting ist lange bekannt. Erstmals 1991 konnte die Freisetzungskinetik versus antimikrobiell Effizienz gezeigt werden (3) . Dies war bei nicht-vernetzten Polymeren mit Lösemitteln möglich, die nichttoxisch sind und leicht evaporiert werden können. Es können damit sehr hohe Wirkstoff-Konzentrationen in dem Kunststoff erreicht werden (1, 3, 5, 7 bis 9) . Zur Erzielung einer langen Abgabedauer ist insbesondere eine molekular-disperse Verteilung einer im Polymer löslichen Substanz geeignet (7). Oberflächlich gebundene Wirkstoffe sind weniger effizient (3, 8).

Eine weitere Möglichkeit zur Herstellung der erfindungsgemäßen Medizinprodukte besteht darin, pharmazeutisch aktive Gefäßprothesen in Form von Schläuchen oder dünnen Röhrchen durch Erspinnen von Fasern aus Polymerlösungen (spezielle Form Solvent Casting) mit den oben genannten Wirkstoffen und Transportieren und Ablegen der Fasern auf einer stabförmigen Unterlage zu einem Vlies herzustellen. Hierbei können Polyamide, Polyacrylate und Polyurethane mit lipophilen oder hydrophilen Substanzen vereinigt werden.

Weitere Möglichkeiten zum Einbringen oder Befüllen von Kunststoffen mit pharmazeutischen Wirkstoffen bestehen in der Extrusion oder im thermischen Spritzguß von Polymeren. Hierbei ist es nur möglich, Wirkstoffe mit Polymeren zu kombinieren, deren Schmelzpunkte und thermische Belastbarkeit ähnlich hoch sind. Die Kombination von Wirkstoffen mit Kunststoffen wie Polyurethan, Silikon, Polyethylentherephthalat, Polyethylen und anderen hochschmelzenden Polymeren ist nur mit Wirkstoffen möglich, die einen ähnlich hohen Schmelzpunkt (bzw. thermische Stabilität) haben. Dies ist beispielsweise für Gentamicinsulfat gegeben. Niedriger schmelzende Kunststoffe für die Schmelzextrusion und den Spritzguß können dabei verwendet werden. Vorzugsweise sind es Polymere, welche in einem Bereich von 100°C ± 30% thermoplastisch verarbeitbar sind. Weiterhin ist es möglich, den Schmelzpunkt von Polymeren mittels CO₂ Begasung bei Extrusion zu senken und somit thermolabile Stoffe hinzuzugeben.

Die Substanzen-Gruppe A umfaßt insbesondere schwach lösliche Salze oder Wachse. Beispiele für solche gering pharmakologisch wirksame Komponenten, welche sehr langsam in Körperflüssigkeiten abgegeben werden, sind beispielsweise nicht-ionische Surfactants wie Acetoylglyceride, Diacetyglyceride, Monoglyceride Diethylen-Glycol-Monostearat, Ethylen-Glycol-Monostearat, Glycol-Monostearat, Verbindungen von Monolaurylethern, Mono-Di-Glyceride, Propylen-Glycol-Monostearat; Sorbit-Monostearat, -Palmitat,-Sesquioleat,-tridecanat,-Tristearat, ionische Surfactants, Hyaluronsäurederivate und Phospholipide. Diese Substanzen können gegebenenfalls die Biokompatibilität eines Medizinproduktes verbessern. Bevorzugt ist für Subtanz A jedoch ein Wirkstoff.

Soweit die Substanz A antimikrobielle Eigenschaften haben soll, hat es sich gezeigt, daß Rifampicin intrazellulär Bakterien penetriert und eine effiziente Substanz gegen Biofilme darstellt (2). Auch andere Ansamycinderivate mit ähnlichen Eigenschaften sind denkbar (Rifamycin, Rifapentin). Vorzugsweise sind antimikrobielle Substanzen mit lipophilen Eigenschaften genannt, welche auch klinischen Nutzen gefunden haben für sogenannte "Difficult to Treat Infections". Denkbar sind grundsätzlich alle antimikrobiell wirksamen Gruppen wie lipophile Vertreter aus der Gruppe der Aminoglykoside, aus der Gruppe der Cephalosporine und darauf bezogenen Betalactame, des Chloramphenicols, Lincosamiden, Makroliden, Penicillinen, Chinolonen, Sulfonamiden, Tetracyclinen, ausgenommen der Kombination Tetracyclin-Minocyclin. Vorzugsweise lipophile Antibiotika sind Benzathin, Phenoxymethylpenicillin, Chloramphenicol, Chlortetracyclin, Ciprofloxacin-Betain, Ciprofloxacin, Clarithromycin, Clindamycin-Palmitathydrochlorid, Trimethoprim, Erythromycin-2-Acetat, -Stearat; Erythromycin-Estolat, Erythromycin-Ethylsuccinat-, Erythromycin-Glutamat, -Laktopropionat, -Stearat, Fusidinsäure, vorzugsweise freie Fusidinsäure, Gramicidin, Mupirocin, lipophile Vertreter der Imidazolreihe wie Econazol, Itraconazol, Clotrimazol und andere, Pristinamycin, Rifabutin, Rifapentin, Rifampicin, Silbersulfadiazin, ausgenommen in der Kombination Silbersulfadiazin-Chlorhexidin, Sparfloxacin, Teicoplanin bzw. Remoplanin, Pristinamycin wegen des lipophilen Alkylrestes und Tyrothricin wegen der Wasserunlöslichkeit.

Besonders erwähnenswert sind Miconazol-Salizylat, Sulfosalicyl-Miconazol bzw. Miconazol-Acetylsalicylat als lipophile Komponente, welche aus der Miconazol-Base präpariert werden können. Diese Substanzen sind vorteilhaft, weil sie antimikrobiell und antithrombogen wirksam sind. Andere antimikrobiell wirksame Substanzen umfassen lipophile Salze des Silbers. Silbersalze in der anorganischen Form sind äußerst hydrophil, diffundieren schlecht in Kunststoffen und der Fachmann erhält somit nicht das gewünschte Slow-Release-System. Lipophile Salze oder Komplexe des Silbers beinhalten die Kombination mit sauren Antibiotika wie z.B. Fusidinsäure oder Mupirocin oder Salicylsäure oder auch einfach nur die Addition von beispielsweise längerkettigen Fettsäuren ab C₂ (= Acetat) bis C_{∞} (= unendlich). Solche lipophilisierten Silbersalze können somit besser diffundieren. Möglich ist auch die Kombination des Antiseptikums 8-Chinolinol, welches Ag⁺ Ionen komplexieren kann und als Ag-Chinolinol schwer wasserlöslich ist. Möglich ist auch die Kombination von Silber (Ag⁺) mit lipophilen Antithrombotika, welche einen pKa-Wert im sauren Bereich aufweisen. Zusätzlich ist es möglich, Silber zu kombinieren mit lipophilen Komplexbildnern wie EDTA und anderen. Dazu gehören Ethylendiamin-Tetraacetat, IGTA, DTPA (Diethyltriaminpentansäure), DMSA, Deferoximin, Dimercaprol, Diethylentetramin. Prinzipiell sind alle Chelate und Salze möglich, welche Silber zu lipophilen Komponenten komplexieren können.

Möglich ist auch die Kombination mit Antiöstrogenen, Antigestagenen, Gestagen oder Östrogen. Hierbei ist es sinnvoll, Blades, welche häufig aus mit Sexualhormonen dotierten Silikonschnitten bestehen und subkutan implantiert werden, zu modifizieren. Diese erreichen durch kontrollierte Freisetzung die gewünschte Antifertilitätskontrolle. Diese Implantate können zudem noch zusätzlich mit antimikrobiellen Wirkstoffen geschützt werden, um Infektionen vorzubeugen.

Auch Wundauflagen aus Polyurethan oder Silikon können beispielsweise mit Ag-Salizylat und einem anderen lipophilen Zusatz modifiert werden.

Weiterhin ist es möglich, durch einen lipophilen Zusatz in Kollagenvliesen die Freisetzung von hydrophilen Antibiotika oder anderen pharmazeutisch wirksamen Substanzen zu beeinflußen. Zudem ist es möglich, auch die Freisetzung von Desinfektionsmitteln wie Chlorhexidin, Octenidin, Polyhexanid und Irgasan für den urologischen Gebrauch zu verzögern und eine länger anhaltende Wirkung zu erreichen. Ferner ist es auch möglich, mit einem lipophilen Zusatz die Freisetzung von antineoplastischen Reagenzien wie z.B. Methotrexat aus Knochenzement zu verlangsamen. Zudem ist es auch möglich, mit einem lipophilen Zusatz Prostaglandine verlangsamt freizusetzen.

Als pharmazeutische Wirkstoffe, insbesondere als eine Substanz A können weiterhin Analgetika wie Auranofin, Benorylat, Diclofenac (Säure) Diflunisal, Flurbiprofen, Ibuprofen, Indomethacin, Ketoprofen, Naproxen, Phenylbutazon und Lokal-Anästetika wie Amethocain, Benzocain und Butylaminobenzoat verwendet werden. Insbesondere weiche oder harte Kontaktlinsen können mit lipophilen Antiphlogistika und Lokal-Anästetika wie Lidocain und Xylocain modifiziert werde.

Implantate für den Stimmritzenersatz bei Kehlkopftumoren können vorzugsweise mit Imidazolen oder Nystatin beschichtet werden.

Androgene und anabole Steroide können bei Gonadenstörungen oder Patienten mit verzögertem Wachstum bzw. Pubertät , bzw. bei Frauen mit Brustkrebs eingesetzt werden. Östrogene werden bei Östrogenmangel, Kontrazeption und menopausalen bzw. postmenopausalen Mangelzuständen lokal appliziert, bei Männern mit Neoplasien der Prostata und bei Frauen mit Brustkrebes. Progesterone werden bei Menstruationsstörungen kombiniert mit Östrogen eingesetzt. Progesterone werden zudem bei endometrialen Hyperplasien und Krebs eingesetzt, sowie bei der hormonellen Kontrazeption beispielsweise als Polymer-Wirkstoffdepot.

Verlangsamte Freisetzungen sind beispielsweise mit folgenden schwer wasserlöslichen Wirkstoffen möglich: Algestone, Acetophenid, Allgyloestrenol, Broperestiol, Chlormadinone-Acetat, Chlortrianisen, Cyproteron, Danzol, Dinoestrol, Drostanolen Propionat, Dihydrogesteron, Ethinoloesteradiol, Ethinyloestradiol, Ethisteron, Ethyloestrenol, Ethynoldiazetat, Fluoxymesteron, Hydroxyprogesteron-Hexanat, Lynoestrinol, Medroxyprogesteronazetat, Megestrolactetat, Metandienon, Methyltestosteron, Norethisteronazetat, Östradiol und die Derivate des Östradiols wie Östradobenzoat-Cypionat, -Dipropionat, -Enantat, -Hexahydrobenzoat - Phenylpropionat-, Undekanat,- Valerat und dekonjugierten Östrogenen wie die veresterten Östrogene, Östrone u.a. aus der Gruppe der Androgene, anabolen Steroide, Östrogenen und Progesteronen.

Antithrombotisch wirksame Substanzen sind beispielsweise alle Heparin-Derivate (gegebenenfalls lipophilisiert), Acetylsalicylsäure-Derivate, Ticlopidin, Argatroban (CAS 73963-72-1), Cloricromen (CAS 68206-94-0), Clorindion (CAS 1146-99-2), Nafazatram, Triflusal und das besonders lipophile Dipyridamol.
- **Figur 1:**: Freisetzung Rifampicin-Clotrimazol-Katheter

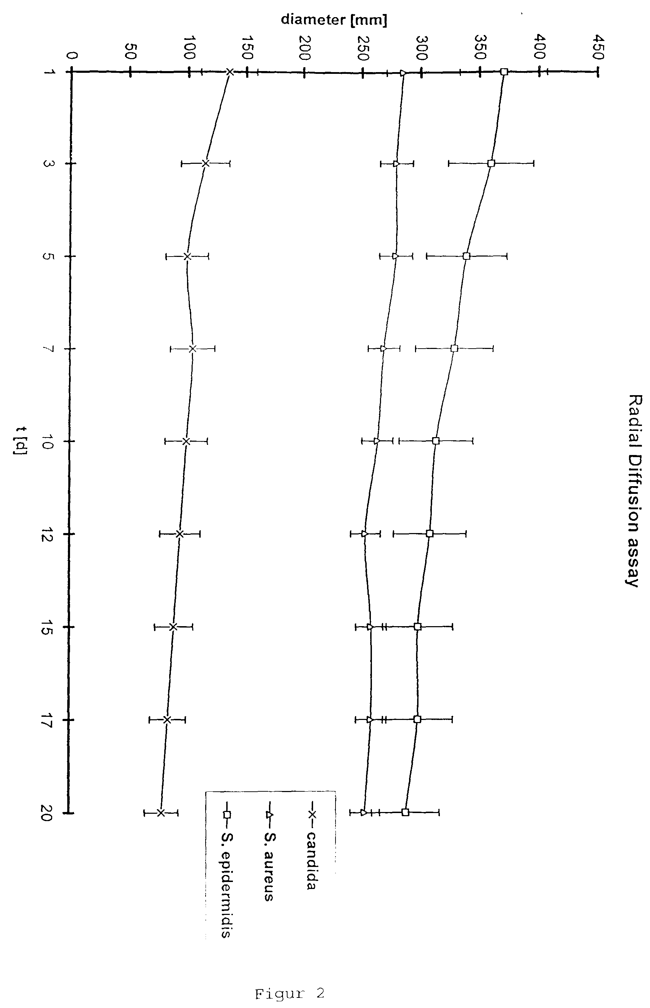

- **Figur 2:**: Antimikrobielle Halbwertzeit im Agar-Diffusionstest gegen unterschiedliche Bakterienspezies

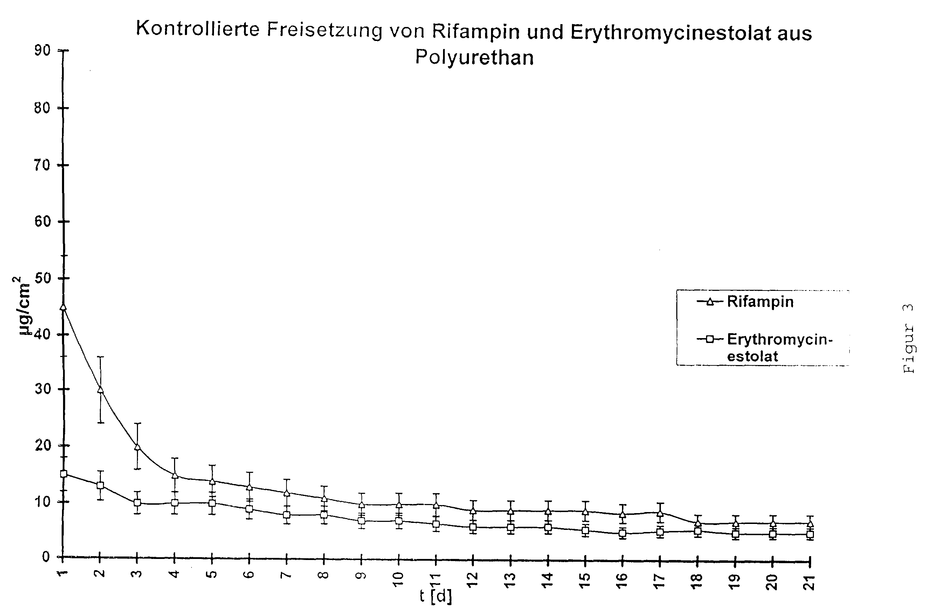

- **Figur 3:**: Freisetzung Rifampicin-Erythromycin-Estolat

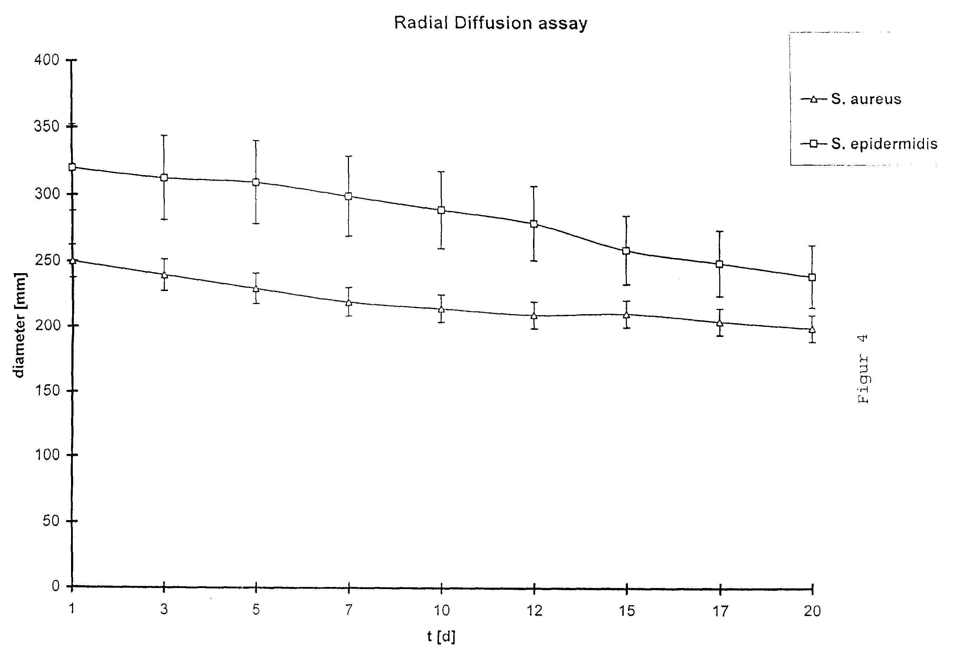

- **Figur 4:**: Antimikrobielle Halbwertzeit im Agardiffusionstest (Staphylokokken)

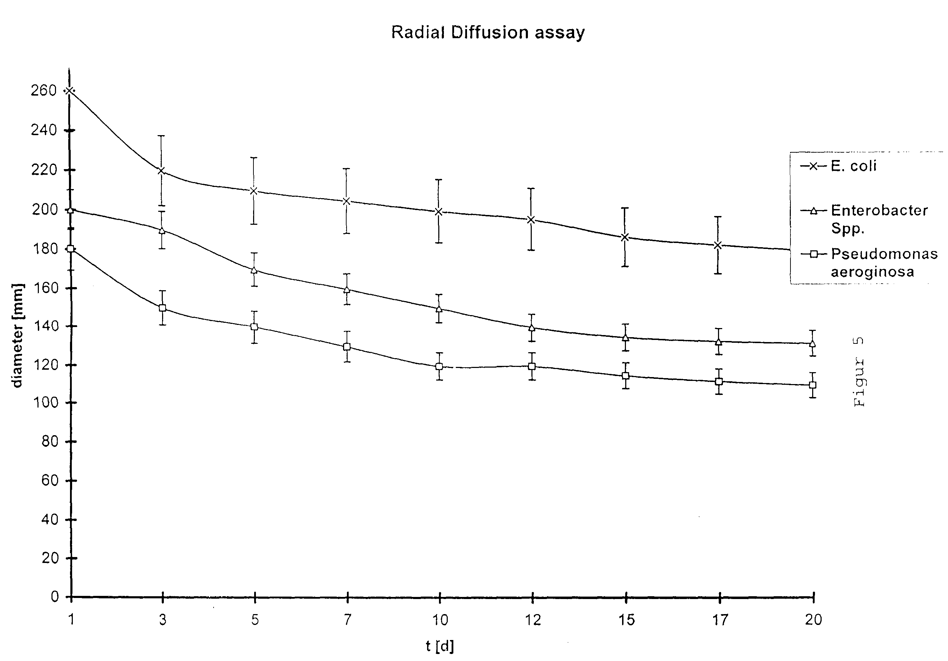

- **Figur 5:**: Antimikrobielle Halbwertzeit im Agardiffusionstest (Enterobactericeae)

Die Erfindung wird anhand der folgenden Beispiele näher erläutert.

### Beispiel 1

Es wird die Herstellung eines Polyurethanfilms beschrieben, welcher Antitumoragentien enthält, welche mittels kontrollierter Freisetzung aus dem medizinischen Kunststoff freigesetzt werden, zu welchem lipophile Substanzen zugesetzt werden, um die Freisetzung zu retardieren. Hier werden Matrix-, Metalloproteinasen bzw. Combretastatin bzw. Vascular-epithelial growth factor bzw. Thalidomid bzw. Squalamin oder SU5416 ein Tyrosinkinaseenzymblocker in einem Verhältnis von 5 Gew. -% zu 5 Gew. -% Miconazolnitrat per Solvent casting in Tetrahydrofuran und Pellethane (10% Pellethane, 1% Substanzen, 89% Solvens) gelöst. Das Polymere wird mit der Substanzmischung in eine Form gegossen, bei 40°C langsam evaporiert, so daß das Lösemittel vollständig abgedampft wird und ein Film entsteht. Die Freisetzung dieser Substanzen, gemessen per HPLC, überschreitet 30 Tage. Nach zwei Tagen erhält man ein steady state release von 2 bis 5 µg pro Substanz pro cm² Polymeroberfläche.

### Beispiel 2

Ein Polyurethankatheter wird in Essigsäureethylester mit 5% (w/w) Rifampicin und 10% (w/w) Clotrimazol bei 30°C 10 Minuten gequollen, so daß die Substanzen in die aufgedehnte polymere Matrix hineindiffundieren können. Danach wird der Katheter aus der Lösung herausgenommen, bei Raumtemperatur 24 Stunden evaporiert, danach bei Unterdruck 48 Stunden und 50°C evaporiert, um sämtliches Restlösemittel aus dem Kunststoff herauszuziehen.

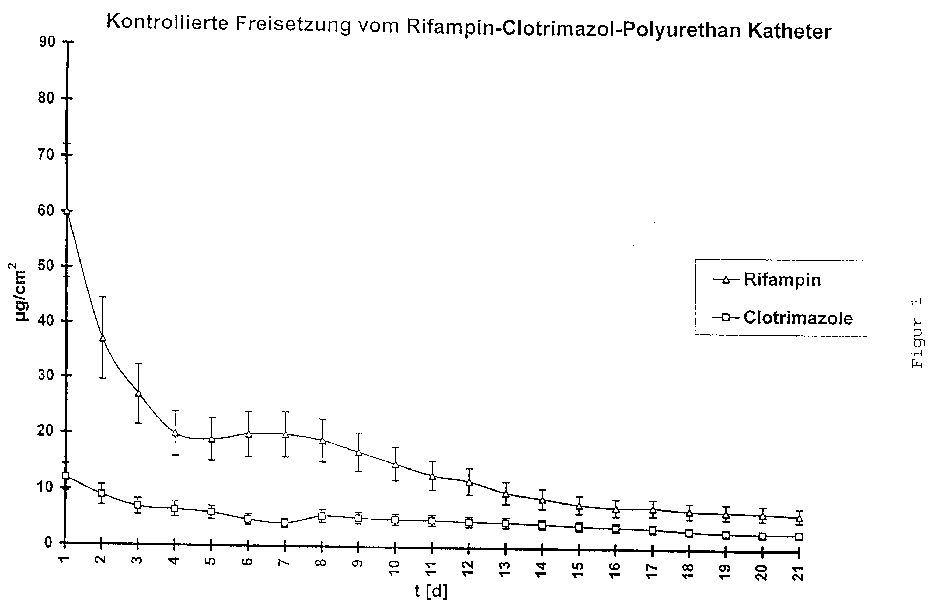

Diese Rifampicin-Clotrimazol-Polyurethanbeschichtung ist in der Lage, mehr als zwei Monate lang effizient Wirkstoff abzugeben. Die Freisetzungskurve von Clotrimazol-Rifampicin ist in Figur 1 dargestellt, in welcher das sehr viel besser wasserlösliche Rifampicin nach dem initialen Burst-Effekt oberflächlich gebundene Wirkstoffe durch den lipophilen Retentionseffekt von Clotrimazol ein ähnliches konstantes Freisetzungsmuster nahezu nullter Ordnung wie Clotrimazol erhält. Dies ist nur dadurch möglich, daß dies sehr schwer wasserlösliche Clotrimazol als Reservoir, Kotransporter und gleichzeitig aber als Retentionsmembran für das hydrophilere Rifampicin fungiert. Dies ist auch mit anderen Substanzen, beispielsweise Fenticonazol oder anderen Imidazol-Derivaten wie Miconazol, Sulfosalicyl-Miconazol, Miconazolnitrat, Miconazolsalicylat bzw. Miconazolacetylsalicylat und anderen Miconazol-Derivaten möglich. Auch andere Imidazol-Derivate, welche fast wasserunlöslich sind, wie Ketoconazol, Itraconazol, Fenticonazol, Econazol und andere sind denkbar. Nach drei Wochen Freisetzungsdauer sind noch > 80% des Wirkstoffs im Implantatmaterial vorhanden. Das Freisetzungsprofil kann auf mehr als drei Monaten approximiert werden.

### Beispiel 3

### Präparationen eines Polyurethan-Katheters mittels Dichlormethan, Rifampicin und einem lipophilen Erythromycinsalz (Erythromycin-Estolat)

Das lipophile Erythromycinsalz ist in wäßrigen Lösungen fast unlöslich im Gegensatz zu dem mäßig wasserlöslichen Rifampicin. Es erfolgte eine Quellung einer 5%igen Rifampicin-, 10%igen Erythromycinlösung mit Pelethane-Kathetern in Dichlormethan 15 min. bei 30°C, Trocknung des gequollenen Katheters 24 Stunden bei Raumtemperatur, dann Evaporieren bei Unterdruck 0,1 mbar, 50°C für 48 Stunden. Hierbei entsteht ein Slow Release-System mit einem Inkorporierungsgehalt von 10 Massenprozent, in welchem nach dem Burst-Effekt die Freisetzung von Rifampicin ähnlich wie in Beispiel 2 parallel zu der langsamen Freisetzung des lipophilen Gentamicinsalzes verläuft. Hierbei wirkt dieses lipophile Erythromycinsalz wiederum als interne Diffusionsmembran.

### Beispiel 4

### Inkorporierung der amphiphilen Chlorhexidin-base und des lipophilen Clindamycin-Plamitats in Polyurethan mittels Solvent Casting

Hierbei wird das Polyurethan in Tetrahydrofuran aufgelöst und in diese Lösung jeweils 5% Chlorhexidin-base und 5% des schwerlöslichen Clindamycinsalzes hinzugegeben. Nach Rühren und Temperaturerhöhung auf 60°C wird diese Lösung in eine Form gegeben, das Lösemittel langsam bei Unterdruck abgedampft und später bei 50°C und Unterdruck vollständig evaporiert. Dieser Wirkstoffenthaltene Polyurethanfilm war in der Lage, mehr als zwei Monate Wirkstoffe freizusetzen, wobei das lipophile Clindamycinsalz die Abgabe von Chlorhexidinbase retardierte und zu einer Freisetzung nahezu nullter Ordnung führte.

### Beispiel 5

### Beschichtung einer Totalendoprothese mit einer Miconazalbase-Rifampicin Mischung

Eine Totalendoprothese, beschichtet mit einer 200 µ dicken, porösen Hydroxy-Apatit-Schicht wurde in eine 5% Rifampicin, 10% Miconazol-Base enthaltende Aceton-Lösung für 5 Minuten gegeben. Daraufhin wurde die Prothese herausgenommen und bei Raumtemperatur 24 Stunden getrocknet. Um die Trägerschicht zu vergrößern, wurde die Prothese noch zweimal 5 Sekunden in das Bad getaucht und getrocknet. Die beschichtete Prothese wurde innerhalb von 20 Tagen in Phosphatpuffer bei 37°C eluiert. Miconazol-Base wirkte hier als Reservoir und Träger für das hydrophilere Rifampicin. Auch die Kombination Rifampicin mit dem schwerlöslichen Ciprofloxacin-betain war 14 Tage freisetzungsaktiv (nicht gezeigt).

### Beispiel 6

### Polyurethan-Harnwegskatheter mit EDTA-Säure / Clindamycin-Beschichtung zur Verhinderung der Inkrustation und Infektion

Eine 10%ige Lösung von Polyurethan in Tetrahydrofuran wurde mit einer 10%igen (bezogen auf das Polymer) Lösung von EDTA und Clindamycin-Palmitat versetzt. Die Lösung wurde wie in Beispiel 4 in eine Form gegeben und evaporiert. Über einen Zeitraum von vier Wochen konnte per HPLC eine Abgabe größer 2 µg/cm² Oberfläche gemessen werden.

### Beispiel 7

### Polyurethan-Harnwegskatheter mit AG-Salizylat und Tioconazol

Eine Lösung aus 90% Tetrahydrofuran, 9% PUR, 0,5% Ag-Salizylat und 0,5% Tioconazol wurde bei 40°C zur Homogenität verührt. Ein Polyurethankatheter wurde sukzessiv für jeweils 20 Sekunden in die Lösung gedippt und getrocknet. Diese Prozedur wurde 5 mal wiederholt. Nach dem zweiten Tag der Elution zeigte der beschichtete Katheter über einen Zeitraum von 30 Tagen eine Freisetzung nahezu nullter Ordnung.

### Literatur

(1) Olanoff LS, Anderson JM, Jones RD: Sustained release of gentamicin from prosthetic heart valves. Trans. Am. Soc. Artif. Intern. Organs 1979; **25:** 334 - 338.
(2) Schierholz JM, Jansen B, Jaenicke L, Pulverer G: In vitro efficacy of an antibiotic releasing silicone ventricle catheter to prevent shunt infection. Biomat. 1994; **15:** 996 - 1000.
(3) Schierholz JM, Jansen B, Steinhauser H, Peters G, Schuhmacher-Perdreau F, Pulverer G: Drug release from antibiotic-containing polyurethanes. New Polymeric Mater 1990; **3**: 61 - 72.
(4) Schierholz JM, Rump AFE, Pulverer G: Drug delivery concepts for efficacious prevention of foreign-body infections. Zbl. Bakt. 1996; 284: 390-401.
(5) Schierholz JM, Rump AFE, Pulverer G: Ciprofloxacin containing polyurethanes as potential drug delivery systems to prevent foreign-body infections. Drug Res. 1997; **47 (I),** 1: 70-74
(6) Schierholz JM: Physico-chemical properties of a rifampicin-releasing polydimethyl-siloxane shunt. Biomat. **17:** 1997
(7) Schierholz JM, Steinhauser H, Rump AFE, Berkels, R, Pulverer G: Controlled release of antibiotics from biomedical polyurethanes: morphological and structural features. Biomat. **18:** 1997
(8) Sherertz RJ, Carruth WA, Hampton AA, Byron MP, Solomon DD: Efficacy of antibiotic-coated catheters in preventing subcutaneous staphylococcus aureus infection in rabbits. J. Inf. Dis. 1993; **167:** 98 - 106.
(9) Solomon DD, Sherertz RJ: Antibiotic releasing polymers. J. Contr. Rel. 1987: 343 - 352.

## Patentansprüche

1. Medizinprodukt umfassend zwei Substanzen, von denen eine erste als Substanz A und eine zweite als Substanz B bezeichnet wird und Substanz A eine Löslichkeit (w/w) in Wasser von 1:1.000 bis 1:1.000.000 aufweist, Substanz B eine höhere Löslichkeit aufweist als Substanz A und zumindest eine der Substanzen A und B ein pharmazeutischer Wirkstoff ist,
ausgenommen
Medizinprodukte enthaltend TDMAC/Minocyclin/EDTA, TDMAC/ Chlorhexidin/Silbersulfadiazine oder TDMAC/Minocyclin/Rifampicin.

2. Medizinprodukt gemäß Anspruch 1, dadurch gekennzeichnet, daß die Substanz A ein pharmazeutischer Wirkstoff ist.

3. Medizinprodukt gemäß Anspruch 1 und/oder 2, dadurch gekennzeichnet, daß die Substanz B ein pharmazeutischer Wirkstoff ist.

4. Medizinprodukt gemäß mindestens einem der Ansprüche 1 bis 3, dadurch gekennzeichnet, daß das Medizinprodukt aus einem Material besteht, daß ausgewählt wird aus Siloxan, Polyurethan, Acrylaten, Polycarbonaten, Cellulose, Cellulose-Derivaten, Polymeren aus Tetrafluorethylen und Polyethylentherephthalat und Hydrogele.

5. Medizinprodukt gemäß mindestens einem der Ansprüche 1 bis 4, dadurch gekennzeichnet, daß die Substanz A ausgewählt wird aus nicht-ionische Surfactants wie Acetoylglyceride, Diacetyglyceride, Monoglyceride Diethylen-Glycol-Monostearat, Ethylen-Glycol-Monostearat, Glycol-Monostearat, Verbindungen von Monolaurylethern, Mono-Di-Glyceride, Propylen-Glycol-Monostearat; Sorbit-Monostearat, -Palmitat, -Sesquioleat, -tridecanat, -Tristearat, Phospholipide, Hyaluronsäurederivate, lipophile Vertreter aus der Gruppe der Aminoglykoside, aus der Gruppe der Cephalosporine und darauf bezogenen Betalactame, des Chloramphenicols, Lincosamiden, Makroliden, Penicillinen, Chinolonen, Sulfonamiden, Tetracyclinen, ausgenommen der Kombination Tetracyclin-Minocyclin, lipophile Antibiotika wie Benzathin, Phenoxymethylpenicillin, Chloramphenicol, Chlortetracyclin, Ciprofloxacin-Betain, Ciprofloxacin, Clarithromycin, Clindamycin-Palmitathydrochlorid, Trimethoprim, Erythromycin-2-Acetat, -Stearat; Erythromycin-Estolat, Erythromycin-Ethylsuccinat-, Erythromycin-Glutamat, -Laktopropionat, -Stearat, Fusidinsäure, vorzugsweise freie Fusidinsäure, Gramicidin, Mupirocin, lipophile Vertreter der Imidazolreihe wie Econazol, Itraconazol, Clotrimazol, Pristinamycin, Rifabutin, Rifapentin, Rifampicin, Silbersulfadiazin, Sulfosalicyl-Miconazol, Miconazol-Salizylat bzw. Miconazol-Acetylsalicylat, lipophile Salze des Silbers, DMSA, Deferoximin, Dimercaprol, Diethylentetramin, Antiöstrogenen, Antigestagenen, Gestagen oder Östrogen, Algestone, Acetophenid, Allgyloestrenol, Broperestiol, Chlormadinone-Acetat, Chlortrianisen, Cyproteron, Danzol, Dinoestrol, Drostanolen Propionat, Dihydrogesteron, Ethinoloesteradiol, Ethinyloestradiol, Ethisteron, Ethyloestrenol, Ethynoldiazetat, Fluoxymesteron, Hydroxyprogesteron-Hexanat, Lynoestrinol, Medroxyprogesteronazetat, Megestrolactetat, Metandienon, Methyltestosteron, Norethisteronazetat, Östradiol und die Derivate des Östradiols wie Östradobenzoat-Cypionat, -Dipropionat, -Enantat, -Hexahydrobenzoat -Phenylpropionat-, Undekanat,- Valerat und dekonjugierten Östrogenen wie die veresterten Östrogene, Östrone u.a. aus der Gruppe der Androgene, anabolen Steroide, Östrogenen und Progesteronen, Heparin-Derivate (gegebenenfalls lipophilisiert), Acetylsalicylsäure-Derivate, Ticlopidin, Argatroban (CAS 73963-72-1), Cloricromen (CAS 68206-94-0), Clorindion (CAS 1146-99-2), Nafazatram, Triflusal und Dipyridamol.

6. Medizinprodukt gemäß mindestens einem der Ansprüche 1 bis 5, dadurch gekennzeichnet, daß die Substanz B ausgewählt wird aus Phospholipiden und Hyaluronsäurederivaten, nicht-ionische Surfactants wie Acetoylglyceride, Diacetyglyceride, Monoglyceride Diethylen-Glycol-Monostearat, Ethylen-Glycol-Monostearat, Glycol-Monostearat, Verbindungen von Monolaurylethern, Mono-Di-Glyceride, Propylen-Glycol-Monostearat; Sorbit-Monostearat, -Palmitat, -Sesquioleat, -tridecanat, -Tristearat und deren hydrophilen Derivaten, lipophile und hydrophile Vertreter aus der Gruppe der Aminoglykoside, aus der Gruppe der Cephalosporine und darauf bezogenen Betalactame, des Chloramphenicols, Lincosamiden, Makroliden, Penicillinen, Chinolonen, Sulfonamiden, Tetracyclinen, ausgenommen der Kombination Tetracyclin-Minocyclin, lipophile Antibiotika wie Benzathin, Phenoxymethylpenicillin, Chloramphenicol, Chlortetracyclin, Ciprofloxacin-Betain, Ciprofloxacin, -Betain, -HCl; Clarithromycin, Clindamycin-Palmitathydrochlorid und hydrophile Vertreter des Clindamycin, Trimethoprim, Erythromycin-2-Acetat, -Stearat; Erythromycin-Estolat, Erythromycin-Ethylsuccinat-, Erythromycin-Glutamat, -Laktopropionat und die hydrophilen Vertreter des Erythromycin, -Stearat, Fusidinsäure, vorzugsweise Natrium-Fusidinsäure, Gramicidin, Mupirocin, lipophile und hydrophile Vertreter der Imidazolreihe wie Econazol, Itraconazol, Clotrimazol und deren hydrophile Vertreter, Pristinamycin, Rifabutin, Rifapentin, Rifampicin und anderen hydrophilen Staphylokokken wirksame Antibiotika, Silbersulfadiazin, Sulfosalicyl-Miconazol, Miconazol-Salizylat bzw. Miconazol-Acetylsalicylat, lipophile Salze und hydrophile Salze des Silbers, EDTA, EDTA-Sibler-Komplex, Ethylendiamin-Tetraacetat, IGTA, DTPA (Diethyltriaminpentansäure), DMSA, Deferoximin, Dimercaprol, Diethylentetramin, Antiöstrogenen, Antigestagenen, Gestagen oder Östrogen, Algestone, Acetophenid, Allgyloestrenol, Broperestiol, Chlormadinone-Acetat, Chlortrianisen, Cyproteron, Danzol, Dinoestrol, Drostanolen Propionat, Dihydrogesteron, Ethinoloesteradiol, Ethinyloestradiol, Ethisteron, Ethyloestrenol, Ethynoldiazetat, Fluoxymesteron, Hydroxyprogesteron-Hexanat, Lynoestrinol, Medroxyprogesteronazetat, Megestrolactetat, Metandienon, Methyltestosteron, Norethisteronazetat, Östradiol und die Derivate des Östradiols wie Östradobenzoat-Cypionat, -Dipropionat, -Enantat, -Hexahydrobenzoat -Phenylpropionat-, Undekanat,- Valerat und dekonjugierten Östrogenen wie die veresterten Östrogene, Östrone u.a. aus der Gruppe der Androgene, anabolen Steroide, Östrogenen und Progesteronen sowie alle hydrophilen Vertreter der Hormone, Heparin-Derivate (gegebenenfalls hydrophilisiert oder lipophilisiert), Acetylsalicylsäure-Derivate, Ticlopidin, Argatroban (CAS 73963-72-1), Cloricromen (CAS 68206-94-0), Clorindion (CAS 1146-99-2), Nafazatram, Triflusal und Dipyridamol.

7. Medizinprodukt gemäß mindestens einem der Ansprüche 1 bis 6, dadurch gekennzeichnet, daß Substanz A und B ausgewählt sind aus Clindamycin/Rifampicin, Tyrothricin/Rifampicin, Hydropregestron-Hexanat/Rifampicin, Clotrimazol/EDTA-Säure, Erythromycin-Stearat/Gentamycinsulfat.

8. Medizinprodukt gemäß mindestens einem der Ansprüche 1 bis 7, dadurch gekennzeichnet, daß der Wirkstoff durch kovalente oder nicht-kovalente Modifikation, wie Veresterung, Etherbildung, Acetal-, Halbacetalbildung, Ringbildung, Adduktbildung, Komplexbildung und/oder Salzbildung mit einem lipophilen Gegenion lipophilisiert wurde.

9. Medizinprodukt gemäß mindestens einem der Ansprüche 1 bis 8, dadurch gekennzeichnet, daß sich der pharmazeutische Wirkstoff intrazellulär in Bakterien und/oder Thrombozyten anreichern kann.

10. Medizinprodukt gemäß mindestens einem der Ansprüche 1 bis 9, dadurch gekennzeichnet, daß das Medizinprodukt eine Augenlinse, ein Katheter, eine Gefäßprothese, eine Endoprothese, ein chirurgisch antimikrobieller Wirkstoffträger wie ein Kollagenvlies, Stents, Blades, Knochenzement, metallische Endoprothesen, CAPD-Katheter, Wundauflagen, gesprühte Polyurethanvliese, Drainage ist.

11. Verfahren zur Herstellung von Medizinprodukten gemäß mindestens einem der Ansprüche 1 bis 10, dadurch gekennzeichnet, daß sie über Quellung, Solvent Casting, Wirkstofflackierung, Extrusion und/oder Spritzguß von Polymeren wie PUR, SIR, PET und/oder durch einen Wirkstoffüberzug mit oder ohne Träger (wie Polymere aus Polylactit, Polyorthoester, Polyethylenglykolen, anderen bioresorbierbaren Polymeren und nicht-resorbierbaren Polymeren) bei metallischen Endoprothesen erhalten werden.

12. Verfahren zur Steuerung der Freisetzung einer Substanz B aus einem Medizinprodukt, dadurch gekennzeichnet, daß Substanz B zusammen mit einer Substanz A in das Medizinprodukt gebracht wird, wobei Substanz A eine Löslichkeit (w/w) in Wasser von 1:1.000 bis 1:1.000.000 aufweist, Substanz B eine höhere Löslichkeit aufweist als Substanz A und zumindest eine der Substanzen A und B ein pharmazeutischer Wirkstoff ist,
